Europäisches Patentamt

European Patent Office

Office européen des brevets

(19)

(11) Publication number: **0 254 272 B1**

# EUROPEAN PATENT SPECIFICATION

(12)

(45) Date of publication of patent specification: **13.04.94** (51) Int. Cl.⁵: **A61K 31/10**

(21) Application number: **87110551.6**

(22) Date of filing: **21.07.87**

(54) The use of probucol for preventing and treating heart diseases.

(30) Priority: **25.07.86 DE 3625279**

(43) Date of publication of application:
**27.01.88 Bulletin 88/04**

(45) Publication of the grant of the patent:
**13.04.94 Bulletin 94/15**

(84) Designated Contracting States:
**AT BE CH DE ES FR GB GR IT LI LU NL SE**

(56) References cited:
**EP-A- 0 212 310**
**US-T- 960 003**

**ARTERY, vol. 10, no. 1, 1982, pages 22-34;
R.E. TEDESCHI et al.: "Safety and effective-
ness of probucol as a cholesterol lowering
agent"**

**J. OCCUP. MED., vol. 23, no. 3, 1981, pages
202-209; G.C. TIMMIS: "Medical management
of ischemic heart disease"**

**ARTERY, vol. 10, no. 1, 1982, pages 35-43;
T.A. MIETTINEN et al.: "Clinical experience
with probucol with special emphasis on
mode of action and long-term treatment"**

(73) Proprietor: **MERRELL DOW PHARMACEUT-
ICALS INC.**
**2110 East Galbraith Road**
**Cincinnati Ohio 45215-6300(US)**

(72) Inventor: **Berg, Aloys, Dr.**
**Medizinische Universitätsklinik**
**Hugstetter Str. 55**
**D-7800 Freiburg(DE)**

(74) Representative: **VOSSIUS & PARTNER**
**Postfach 86 07 67**
**D-81634 München (DE)**

THE AMERICAN JOURNAL OF CARDIOLOGY, vol. 49, / 4II, 1982, page 914; M.J. BARBER et al.: "Effect of probucol on left ventricular myocardial blood flow during sympathetic nerve stimulation and maximal coronary vasodilation"

JAMA, vol. 254, no. 15, 1985, pages 2097-2102; T.A. MIETTINEN et al.: "Multifactorial primary prevention of cardiovascular diseases in middle-aged men"

CHEM-ENG. NEWS, vol. 60, no. 28, 12th July 1982, pages 26-38; H.J. SANDERS: "New drugs for combating heart disease"

EP 0 254 272 B1

## Description

The invention relates to the use of probucol for preventing and treating heart diseases, wherein a reduced myocardial oxygen consumption is desirable or required wherein the heart disease to be treated is independent of hyperlipidemia. Examples of such diseases are cardiomyopathy of various origins, the hyperkinetic heart syndrome and coronary heart disease, including coronary insufficiency and its concomitant symptoms or secondary diseases.

Concomitant symptoms of coronary insufficiency may be angina pectoris attacks, a temporary insufficiency of the myocardial contractility associated with a lessening in the force of the heart action, arrhythmias etc. Secondary diseases of coronary insufficiency may be tissue necroses with subsequent irreversible heart insufficiency, myocardial infarct, irreversible arrhythmias, etc.

Probucol is administered in doses of 1000 mg/day to treat certain metabolic disorders which are accompanied by increased serum cholesterol levels; see US Patent 3,862,332.

The US Defensive Publication No. 960003 contains test results relating to signs of histopathological injuries, signs of cardiomyopathy, signs of tissue necrosis and signs of tissue adhesion in the case of experimentally induced trauma i.e. mechanical trauma (operation), chemical trauma (isoproterenol, cobalt, chloride) and/or pathological trauma (cardiac infarction, aortic stenosis, congenital cardiomyopathy, nephritis, damage of the liver parenchyma) and their response to probucol in mammals.

Changes in the heart rate, in particular under physical stress, and a positive influence on the regulation of the heart and the blood circulation and the function of the heart are not mentioned. The term "cardioprotective" is used only in connection with histopathological findings but not, as is usual in cardiology, with the function of the heart, the corresponding oxygen consumption and the coronary reserve depending on it.

The publication "ARTERY", 10(1), pages 22-34 (1982) reviews a clinical study about the hypocholesterolemic effect of probucol. On the basis of this study it is suggested that probucol may protect from having a myocardial infarction.

In "J. Occup. Med." 23(3), pages 202-209 (1981) the medical management of ischemic heart disease is reviewed.

On page 205 under the heading "Therapy to Maintain Myocardial Oxygen Supply (Prevention of Coronary artherosclerosis)" the evolution of coronary artherosclerosis is described and its relationship to cholesterol. The use of probucol for lowering cholesterol levels is discussed, to attempt to prevent the inexorable progression of artherosclerosis and thereby the maintenance of the myocardial oxygen supply.

In the publication "The American Journal of Cardiology", vol. 49, page 914 (1982) an arrhythmic action of probucol is described. Moreover, the authors of this document conclude that probucol has unfavorable effects on the coronary vasculature.

In "JAMA", vol. 254 (15), pages 2097-2102 (1985) probucol is described as a hypolipidemic agent. Its usefulness in reducing high plasma cholesterol levels, one of the risk factors of coronary heart disease in a multifactorial treatment is discussed.

In "Chem. Erg. News", vol. 60 (28) (1982) the pathophysiology of artherosclerosis is described, in particular the narrowing of the blood vessels and thereby the decrease in blood flow and decrease in oxygen supply. Moreover, probucol is described here as an anticholesterol agent.

Surprisingly, probucol has been found to have an advantageous effect on the function and performance capacity of the heart. When considering the result of ergometric testing in judging the size of the heart, clear signs can be found for the favourable influence of probucol on the regulation of the heart and the circulation in addition to its lowering of the increased serum cholesterol values. This mode of action is reflected in the changes during exercise of the heart rate, the power and oxygen utilization and the cardiac work.

Patients and Method:

To examine the mode of action of probucol on the regulation of the heart and the blood circulation, 16 normal, clinically healthy males aged 20 to 40 years were administered probucol after stress and examined for stress-induced changes in the heart and the blood circulation. For this purpose, the results of six defined ergometries (gradual bicycle ergometry to the subjective maximum strain whilst lying down, loads each increased by 50 watt and applied for 3 minutes, starting load 100 watt, EC gramm at the end of each stress minute) were collected in each case. The data from 2 tests with medication (2x500 mg probucol, duration of medication 4 and 8 weeks) were contrasted in an examination of the statistical significance (student's t test in paired data) with the findings of 4 control tests (4 weeks and immediately before starting the medication,

3

4 and 12 weeks after discontinuing the medication).

While the findings for the individual body weight and the maximum performance capacity, the action of the heart and the heart volume at rest, the regulation and utilization of the carbohydrates before, during and after exercise remain unchanged, the administration of probucol was found to specifically influence

- the heart rate under exercise (see Table I)
- the power (see Table II)
- the oxygen utilization (see Table III) and
- the cardiac work under exercise (see Table IV).

Starting from almost identical heart rates in repose and comparable heart rates at moderate strain (100 watt corresponding to 40% of the maximum performance capacity, the stress-induced increase in the rate compared to the results in the control test can be reduced by 10 beats (-13%) in the submaximum range and by 15 beats (-20%) at maximum strain; this effect can already be significantly demonstrated by about 10% at intensities of around 60% of the maximum performance capacity. Moreover, without restricting the maximum performance capacity under medication, the cardiac work, which can be measured in the product of heart rate and systolic blood pressure, is lowered by about 10%. The performance capacity of the heart, judged by the quotient formed from the power or oxygen utilization, is likewise significantly increased by about 7%.

These effects of the test substance must be acknowledged to be of so far unknown, therapeutic importance for a risk population suffering from coronary diseases. The results show that the administration of probucol makes it possible to widen the regulatory range of the heart and the blood circulatory system in submaximum and maximum performance without simultaneously reducing the maximum performance capacity. In view of the established relationship between the blood pressure heart rate product and the oxygen consumption of the heart on the one hand and the oxygen consumption of the heart and the threshold of pain of angina pectoris on the other hand, the lowering of the blood pressure heart rate product by probucol means an improvement of the coronary reserve, and, in the case of patients suffering from coronary diseases, it also means the improvement of the performance capacity limited by symptoms. It is for these very risk patients with coronary heart diseases suffering from dyslipoproteinemia and coronary angiopathy, that it is desirable to lower the cardiac work and the myocardial oxygen consumption, as an improvement of the coronary reserve under strain can be expected to result in a lessening of the ischemic reaction and reduced arrhythmias. Compared to other preparations which reduce the heart rate and the heart action (for instance betablockers), probucol should be especially highlighted in that it exerts this cardioprotective effect, without simultaneously affecting the maximum performance capacity and the contractility reserve of the heart, the latter being perceivable from the former. This is all the more important for persons who can be assumed to already have a lower contractility reserve, for in this case the substance would have to be held to show an undesirable side effect, if its negatively inotropic property, which acts independently, appeared at the same time as the lowering of the heart rate.

On the basis of the results that are available on the influence of probucol on the regulation of the heart and the blood circulation, the following effects not known so far and differing from other substances are to be highlighted.

1. With defined intensities, the individual heart rate is markedly lower as from about 60% of the maximum performance capacity, while the maximum performance remains unchanged. A favourable influence is exerted on the parameters of the working heart (power, oxygen utilization, blood pressure heart rate product), while there is no sign of a lower contractility reserve.

2. The regulation of parameters of the heart and the blood circulation at rest remains unchanged; there are no signs of undesired blocking of the sympathoadrenergic tonus

3. Nor are there any indications that the reduced action of the heart is coupled with a simultaneous increase in the peripheric muscular strain or fatigue (glucose and lactate behaviour) or an undesired inhibitory effect on the peripheric energy supply (glucose utilization, glycogenolysis).

Pharmaceutical compositions containing probucol as the active ingredient can be prepared in the manner described in US Patent 3,862,332. The pharmaceutical compositions can be administered orally, or parenterally. The oral route is preferred. Typical examples of dosage unit forms are tablets and injection preparations. The dose depends on the severity of the patient's illness, on his age and weight. The daily dose may be about 2 to 20 mg, preferably 4 to 15 mg of the active ingredient per kg of bodyweight.

## Table I

### Heart rate under exercise

#### 1a: Heart rate at rest

| Test | Average value | Standard deviation | Minimum | Maximum |
|------|--------------|--------------------|---------|---------|
| 1 | 68,19 | 11,96 | 48,00 | 90,00 |
| 2 | 68,69 | 12,12 | 48,00 | 89,00 |
| 3 | 66,19 | 11,34 | 48,00 | 90,00 |
| 4 | 67,50 | 13,36 | 47,00 | 95,00 |
| 5 | 65,81 | 10,17 | 50,00 | 85,00 |
| 6 | 65,75 | 8,39 | 51,00 | 83,00 |

Reference group: test I
tested against:

| Test | AV (Diff.) | Standard deviation | F.grade | : t-Value | p < |
|------|-----------|--------------------|---------|-----------|-----|
| 2 | 0,50 | 9,60 | 15 | : 0,208 | 0,8377 |
| 3 | -2,00 | 9,19 | 15 | :-0,871 | 0,3976 |
| 4 | -0,69 | 12,19 | 15 | :-0,226 | 0,8245 |
| 5 | -2,38 | 9,67 | 15 | :-0,982 | 0,3417 |
| 6 | -2,44 | 9,74 | 15 | : -1,001 | 0,3325 |

EP 0 254 272 B1

The numbers in this and the following Tables mean:

1 = control test 4 weeks before medication
2 = control test 1 day before medication
3 = examination after 4 weeks of medication
4 = examination after 8 weeks of medication
5 = control test 4 weeks after discontinuing the medication
6 = control test 12 weeks after discontinuing the medication

## Table I - continued

### Ib: Load of 200 watt

| Test | Average value | Standard deviation | Minimum | Maximum |
|---|---|---|---|---|
| 1 | 147,27 | 14,86 | 122,00 | 179,00 |
| 2 | 145,40 | 16,30 | 120,00 | 173,00 |
| 3 | 137,75 | 14,26 | ·111,00 | 162,00 |
| 4 | 137,33 | 14,41 | 117,00 | 164,00 |
| 5 | 141,63 | 15,51 | 118,00 | 174,00 |
| 6 | 143,25 | 15,18 | 108,00 | 173,00 |

Reference group: test 1
tested against

| Test | AV (Diff.) | SD (Diff.) | F.grade | : | t-Value | $p <$ | |
|---|---|---|---|---|---|---|---|
| 2 | -1,87 | 6,98 | 14 | : | -1,036 | 0,3178 | |
| 3 | -10,00 | 5,44 | 14 | : | -7,122 | 0,0000 | *** |
| 4 | -9,93 | 8,08 | 14 | : | -4,764 | 0,0003 | *** |
| 5 | - 4,87 | 9,11 | 14 | : | -2,069 | 0,0575 | |
| 6 | -4,40 | 10,37 | 14 | : | -1,643 | 0,1226 | |

EP 0 254 272 B1

## Table 1 - continued

### Ic: Maximum load

| Test | Average value | Standard deviation | Minimum | Maximum |
|------|---------------|--------------------|---------|---------|
| 1 | 170,13 | 14,92 | 140,00 | 195,00 |
| 2 | 167,56 | 14,50 | 137,00 | 195,00 |
| 3 | 155,31 | 11,01 | 135,00 | 177,00 |
| 4 | 157,25 | 9,66 | 142,00 | 184,00 |
| 5 | 165,00 | 14,03 | 130,00 | 185,00 |
| 6 | 164,63 | 11,52 | 144,00 | 185,00 |

Reference group: test 1
tested against:

| Test | AV (Diff.) | Standard deviation | F.grade | : t-Value | p < | |
|------|-----------|--------------------|---------|-----------|-----|---|
| 2 | -2,56 | 5,56 | 15 | : -1,843 | 0,0852 | |
| 3 | -14,81 | 12,05 | 15 | : -4,919 | 0,0002 | *** |
| 4 | -12,88 | 14,88 | 15 | : -3,462 | 0,0035 | ** |
| 5 | -5,13 | 14,17 | 15 | : -1,446 | 0,1682 | |
| 6 | -5,50 | 10,00 | 15 | : -2,200 | 0,0439 | * |

EP 0 254 272 B1

## Table II

### Power

### IIa: Load of 100 watt

| Test | Average value | Standard deviation | Minimum | Maximum |
|------|---------------|--------------------|---------|---------|
| 1 | 0,96 | 0,15 | 0,79 | 1,35 |
| 2 | 0,96 | 0,10 | 0,82 | 1,11 |
| 3 | 1,00 | 0,15 | 0,84 | 1,37 |
| 4 | 1,00 | 0,14 | 0,78 | 1,32 |
| 5 | 0,97 | 0,10 | 0,81 | 1,18 |
| 6 | 0,96 | 0,12 | 0,85 | 1,35 |

Reference group: test 1
tested against:

| Test | AV (Diff.) | SD (Diff.) | F.grade | : | t-Value | p < |
|------|-----------|-----------|---------|---|---------|-----|
| 2 | -0,01 | 0,09 | 15 | : | -0,365 | 0,7205 |
| 3 | 0,04 | 0,12 | 15 | : | 1,212 | 0,2442 |
| 4 | 0,03 | 0,13 | 15 | : | 1,011 | 0,3282 |
| 5 | 0,00 | 0,09 | 15 | : | 0,077 | 0,9399 |
| 6 | -0,00 | 0,08 | 15 | : | -0,028 | 0,9782 |

EP 0 254 272 B1

EP 0 254 272 B1

Table II - continued

IIb: Load of 200 watt

| Test | Average value | Standard deviation | Minimum | Maximum |
|---|---|---|---|---|
| 1 | 1,37 | 0,14 | 1,12 | 1,64 |
| 2 | 1,39 | 0,16 | 1,16 | 1,67 |
| 3 | 1,47 | 0,16 | 1,24 | 1,80 |
| 4 | 1,47 | 0,15 | 1,22 | 1,71 |
| 5 | 1,43 | 0,16 | 1,15 | 1,70 |
| 6 | 1,41 | 0,16 | 1,16 | 1,85 |

Reference group: test 1
tested against:

| Test | AV (Diff.) | SD (Diff.) | F. grade | : | t-Value | $p <$ | |
|---|---|---|---|---|---|---|---|
| 2 | 0,02 | 0,07 | 14 | : | 1,144 | 0,2718 | |
| 3 | 0,10 | 0,06 | 14 | : | 6,739 | 0,0000 | *** |
| 4 | 0,10 | 0,08 | 14 | : | 4,728 | 0,0003 | *** |
| 5 | 0,05 | 0,10 | 14 | : | 2,014 | 0,0637 | |
| 6 | 0,05 | 0,10 | 14 | : | 1,685 | 0,1141 | |

## Table II - continued

### IIc - Maximum load

| Test | Average value | Standard deviation | Minimum | Maximum |
|------|---------------|--------------------|---------|---------|
| 1 | 1,51 | 0,27 | 1,12 | 2,19 |
| 2 | 1,50 | 0,26 | 1,12 | 2,19 |
| 3 | 1,61 | 0,26 | 1,24 | 2,33 |
| 4 | 1,61 | 0,26 | 1,24 | 2,33 |
| 5 | 1,56 | 0,21 | 1,15 | 1,88 |
| 6 | 1,53 | 0,24 | 1,16 | 2,16 |

Reference group: test 1
tested against:

| Test | AV (Diff.) | Standard deviation | F.grade | : | t-Value | $p <$ | |
|------|-----------|--------------------|---------|---|---------|-------|---|
| 2 | -0,01 | 0,06 | 15 | : | -0,542 | 0,5956 | |
| 3 | 0,10 | 0,06 | 15 | : | 6,192 | 0,0000 | *** |
| 4 | 0,10 | 0,08 | 15 | : | 5,251 | 0,0001 | *** |
| 5 | 0,05 | 0,16 | 15 | : | 1,177 | 0,2575 | |
| 6 | 0,02 | 0,12 | 15 | : | 0,709 | 0,4894 | |

EP 0 254 272 B1

## Table III

### Oxygen Utilization

#### IIIa: Load of 150 watt

| Test | Average value | Standard deviation | Minimum | Maximum |
|---|---|---|---|---|
| 1 | 16,37 | 1,82 | 13,76 | 19,64 |
| 2 | 16,53 | 1,89 | 13,31 | 20,85 |
| 3 | 17,21 | 2,32 | 14,45 | 23,25 |
| 4 | 16,98 | 1,78 | 13,95 | 19,64 |
| 5 | 16,78 | 1,88 | 13,85 | 20,64 |
| 6 | 16,50 | 1,74 | 14,34 | 21,07 |

Reference group: test 1
tested against:

| Test | AV (Diff.) | SD (Diff.) | F.grade | : | t-Value | p < | |
|---|---|---|---|---|---|---|---|
| 2 | 0,17 | 1,08 | 15 | : | 0,612 | 0,5497 | |
| 3 | 0,84 | 1,31 | 15 | : | 2,563 | 0,0216 | * |
| 4 | 0,61 | 1,25 | 15 | : | 1,951 | 0,0700 | |
| 5 | 0,42 | 1,23 | 15 | : | 1,356 | 0,1951 | |
| 6 | 0,13 | 0,91 | 15 | : | 0,589 | 0,5644 | |

EP 0 254 272 B1

EP 0 254 272 B1

## Table III - continued

### IIIb: Load of 200 watt

| Test | Average value | Standard deviation | Minimum | Maximum |
|---|---|---|---|---|
| 1 | 17,75 | 1,78 | 14,47 | 21,23 |
| 2 | 18,03 | 2,08 | 14,97 | 21,58 |
| 3 | 19,00 | 2,03 | 15,99 | 23,33 |
| 4 | 19,05 | 1,96 | 15,79 | 22,14 |
| 5 | 18,49 | 2,02 | 14,89 | 21,95 |
| 6 | 18,28 | 2,08 | 14,97 | 23,98 |

Reference group: test 1
tested against:

| Test | AV (Diff.) | SD (Diff.) | F.grade | : | t-Value | p < | |
|---|---|---|---|---|---|---|---|
| 2 | 0,28 | 0,94 | 14 | : | 1,138 | 0,2741 | |
| 3 | 1,32 | 0,76 | 14 | : | 6,735 | 0,0000 | *** |
| 4 | 1,30 | 1,07 | 14 | : | 4,698 | 0,0003 | *** |
| 5 | 0,65 | 1,24 | 14 | : | 2,010 | 0,0641 | |
| 6 | 0,59 | 1,36 | 14 | : | 1,684 | 0,1143 | |

EP 0 254 272 B1

## Table III - continued

### IIIc: Maximum load

| Test | Average value | Standard deviation | Minimum | Maximum |
|------|---------------|--------------------|---------|---------|
| 1 | 19,05 | 3,19 | 14,47 | 26,83 |
| 2 | 18,99 | 3,08 | 14,62 | 26,83 |
| 3 | 20,35 | 3,07 | 15,99 | 28,62 |
| 4 | 20,37 | 3,02 | 15,99 | 28,62 |
| 5 | 19,66 | 2,42 | 14,89 | 23,28 |
| 6 | 19,34 | 2,74 | 14,97 | 26,50 |

Reference group: test 1
tested against:

| Test | AV (Diff.) | Standard deviation | F.grade | : | t-Value | p < | |
|------|-----------|--------------------|---------|---|---------|-----|---|
| 2 | -0,06 | 0,66 | 15 | : | -0,370 | 0,7164 | |
| 3 | 1,30 | 0,80 | 15 | : | 6,464 | 0,0000 | *** |
| 4 | 1,32 | 1,00 | 15 | : | 5,261 | 0,0001 | *** |
| 5 | 0,61 | 1,97 | 15 | : | 1,231 | 0,2372 | |
| 6 | 0,29 | 1,40 | 15 | : | 0,829 | 0,4199 | |

## Table IV

### Cardiac work

### IVa:_Cardiac_work_at_rest

| Test | Average value | Standard deviation | Minimum | Maximum |
|---|---|---|---|---|
| 1 | 8,11 | 1,53 | 5,04 | 10,80 |
| 2 | 8,39 | 1,81 | 5,28 | 11,13 |
| 3 | 8,07 | 1,66 | 5,76 | 11,31 |
| 4 | 7,91 | 1,83 | 5,17 | 11,40 |
| 5 | 7,54 | 1,63 | 5,25 | 11,05 |
| 6 | 7,81 | 1,51 | 5,87 | 11,21 |

Reference group: test 1
tested against:

| Test | AV (Diff.) | Standard deviation | F.grade | : t-Value | $p <$ |
|---|---|---|---|---|---|
| 2 | 0 ,39 | 1,28 | 14 | : 1,192 | 0,2530 |
| 3 | −0 ,04 | 1,43 | 15 | : −0,109 | 0,9143 |
| 4 | −0 ,20 | 1,69 | 15 | : −0,468 | 0,6464 |
| 5 | −0 ,57 | 1,21 | 15 | : −1,905 | 0,0761 |
| 6 | −0 ,30 | 1,27 | 15 | : −0,949 | 0,3579 |

EP 0 254 272 B1

## Table IV - continued

### IVb: Load of 200 watt

| Test | Average value | Standard deviation | Minimum | Maximum |
|---|---|---|---|---|
| 1 | 27,91 | 4,33 | 18,30 | 38,49 |
| 2 | 27,26 | 3,99 | 20,74 | 36,33 |
| 3 | 25,71 | 4,18 | 19,98 | 33,88 |
| 4 | 24,85 | 4,30 | 19,20 | 34,02 |
| 5 | 24,58 | 4,12 | 18,00 | 33,06 |
| 6 | 25,90 | 3,66 | 19,98 | 34,60 |

Reference group: test 1
tested against:

| Test | AV (Diff.) | SD (Diff.) | F.grade | : t-Value | $p <$ | |
|---|---|---|---|---|---|---|
| 2 | -0,66 | 3,07 | 14 | :-0,828 | 0,4217 | |
| 3 | -2,08 | 3,01 | 14 | :-2,670 | 0,0183 | * |
| 4 | -3,06 | 2,87 | 14 | :-4,124 | 0,0010 | ** |
| 5 | -3,08 | 2,58 | 14 | :-4,622 | 0,0004 | *** |
| 6 | -2,08 | 2,89 | 14 | :-2,779 | 0,0148 | * |

EP 0 254 272 B1

Table IV – continued

IVc: Maximum load

| Test | Average value | Standard deviation | Minimum | Maximum |
|---|---|---|---|---|
| 1 | 35,03 | 4,30 | 26,60 | 40,95 |
| 2 | 34,67 | 4,69 | 24,48 | 41,14 |
| 3 | 31,54 | 4,81 | 23,93 | 44,25 |
| 4 | 31,52 | 3,95 | 23,68 | 41,40 |
| 5 | 31,82 | 4,72 | 20,80 | 42,55 |
| 6 | 30,50 | 6,00 | 12,68 | 39,60 |

Reference group: test 1 tested against:

| Test | AV (Diff.) | SD (Diff.) | F.grade | t-Value | $p \langle$ | |
|---|---|---|---|---|---|---|
| 2 | -0,36 | 2,64 | 15 | -0,551 | 0,5899 | |
| 3 | -3,50 | 3,80 | 15 | -3,685 | 0,0022 | ** |
| 4 | -3,51 | 3,20 | 15 | -4,389 | 0,0005 | *** |
| 5 | -3,22 | 3,64 | 15 | -3,536 | 0,0030 | ** |
| 6 | -4,53 | 6,68 | 15 | -2,716 | 0,0159 | * |

## Claims

1. The use of probucol for the preparation of a medicament for preventing and treating heart diseases by a reduction of myocardial oxygen consumption, wherein the heart disease to be treated is independent of hyperlipidemia.

**2.** An embodiment according to claim 1, characterized in that the disease to be treated is the coronary heart disease, including coronary insufficiency and its consecutive symptoms or secondary diseases.

**3.** An embodiment according to claim 1, characterized in that the disease to be treated is cardiomyopathy of various origins and the hyperkinetic heart syndrom.

**Patentansprüche**

**1.** Verwendung von Probucol zur Herstellung eines Arzneimittels zur Vorbeugung und Behandlung von Erkrankungen des Herzens durch eine Reduzierung des myocardialen Sauerstoffbedarfs, wobei die zu behandelnde Erkrankung des Herzens unabhängig von einer Hyperlipidemie ist.

**2.** Ausführungsform nach Anspruch 1, dadurch gekennzeichnet, daß es sich bei der zu behandelnden Erkrankung um die koronare Herzkrankheit, einschließlich der Koronarinsuffizienz und ihrer Folgeerscheinungen bzw. Folgeerkrankungen handelt.

**3.** Ausführungsform nach Anspruch 1, dadurch gekennzeichnet, daß es sich bei der zu behandelnden Erkrankung um Kardiomyopartie unterschiedlicher Genese und das hyperkinetische Herzsyndrom handelt.

**Revendications**

**1.** Utilisation de probucol pour la préparation d'un médicament destiné à prévenir et traiter les cardiopathies par réduction de la consommation d'oxygène du myocarde, dans laquelle la cardiopathie à traiter n'est pas liée à une hyperlipidémie.

**2.** Mode de réalisation selon la revendication 1, caractérisé en ce que la cardiopathie à traiter est une coronaropathie, comprenant insuffisance coronarienne et ses symptômes consécutifs ou ses maladies secondaires.

**3.** Mode de réalisation selon la revendication 1, caractérisé en ce que la cardiopathie à traiter est une cardiomyopathie d'origines diverses et l'éréthisme cardiovasculaire.